# EUROPÄISCHE PATENTSCHRIFT

(11) **EP 3 664 036 B1**
(45) Veröffentlichungstag und Bekanntmachung des Hinweises auf die Patenterteilung: **17.08.2022**
(21) Anmeldenummer: 19171766.9
(22) Anmeldetag: 30.04.2019
(51) Int. Cl.: G06T 11/00, G16H 30/40, G16H 40/63

(54) **VERFAHREN UND SYSTEM ZUR BERECHNUNG EINER AUSGABE VON EINEM DURCH EINEN TOMOGRAPHEN BEREITGESTELLTEN SCROLLBAREN BILDSTAPEL**
METHOD AND SYSTEM FOR CALCULATING AN OUTPUT OF A SCROLLABLE STACK OF IMAGES PROVIDED BY A TOMOGRAPHER
PROCÉDÉ ET SYSTÈME DE CALCUL D'UNE SORTIE D'UN EMPILEMENT D'IMAGES POUVANT DÉFILER FOURNI À L'AIDE D'UN TOMOGRAPHE

(43) Veröffentlichungstag der Anmeldung: 10.06.2020
(73) Patentinhaber: Siemens Healthcare GmbH, 91052 Erlangen (DE)
(72) Erfinder: Baer-Beck, Matthias, 91054 Erlangen (DE); Faby, Sebastian, 91301 Forchheim (DE); Raupach, Rainer, 91336 Heroldsbach (DE); Ritter, André, 91077 Neunkirchen am Brand (DE)

(56) Entgegenhaltungen:
- WO-A2-2017/091835
- US-A1- 2018 338 741
- SONG HYUNJOO ET AL: "GazeVis: Interactive 3D Gaze Visualization for Contiguous Cross-Sectional Medical Images", IEEE TRANSACTIONS ON VISUALIZATION AND COMPUTER GRAPHICS, IEEE SERVICE CENTER, LOS ALAMITOS, CA, US, Bd. 20, Nr. 5, Mai 2014 (2014-05), Seiten 726-739, XP011543258, ISSN: 1077-2626, DOI: 10.1109/TVCG.2013.271 [gefunden am 2014-03-17]

## Beschreibung

Bei der Darstellung eines von einem Tomographen bereitgestellten scrollbaren Bildstapels für medizinische Untersuchungen ist es häufig wünschenswert, einem Betrachter, beispielsweise einem Radiologen, zusätzliche bzw. detailliertere und/oder weiterführende Informationen für eine Untersuchung zur Verfügung zu stellen. Diese zusätzlichen Informationen können weitere Bildstapel des gleichen medizinischen Falls mit ergänzenden Funktionen sein. Beispielsweise können neben einem Bildstapel einer Computertomographie noch ein weiterer Bildstapel einer Computertomographie mit einer Kontrastmittelphase und/oder funktionelle Informationen (z.B. Perfusion, Ventilation) und/oder auch verschiedenartig rekonstruierte Bildstapel des gleichen Aufnahmedatensatzes von Interesse sein. Beispielsweise können Ventilationskarten berechnet werden. Diese stellen dar, wie gut die Lunge lokal belüftet ist. Diese Ventilationskarten können entsprechend der Belüftung unterschiedlich farblich dargestellt werden. Eine weitere Klasse an zusätzlichen Informationen stellen angezeigte Annotationen am Bildstapel dar.

US 2018/338741 A1 offenbart ein Lungenscreening-Bewertungssystem, welches basierend auf einem Brust-Computertomographie-Scan, der mehrere Querschnittsbilder enthält, betrieben wird.

WO 2017/091835 A2 offenbart eine Betriebsmethode zur Verwendung mit einem auf Magnetresonanztomographie basierenden medizinischen Bildgebungssystem zur automatischen Korrektur von Phasenaliasing.

SONG HYUNJOO ET AL: "GazeVis: Interactive 3D Gaze Visualization for Contiguous Cross-Sectional Medical Images", IEEE TRANSACTIONS ON VISUALIZATION AND COMPUTER GRAPHICS, IEEE SERVICE CENTER, LOS ALAMITOS, CA, US, Bd. 20, Nr. 5, Mai 2014 (2014-05), Seiten 726-739, offenbart eine Methode zur interaktiven 3D-Blickvisualisierung für zusammenhängende medizinische Querschnittsbilder.

Bei im Stand der Technik bekannten Systemen besteht die Herausforderung, die bereitgestellten scrollbaren Bildstapel und die zusätzlichen Informationen derart zu präsentieren, dass der Betrachter einerseits die räumliche Korrelation mit der primären Bildinformation herstellen kann, andererseits jedoch die primäre, möglicherweise für die Untersuchung diagnostisch relevante Bildinformation des scrollbaren Bildstapels aber nicht überdeckt wird. Hierzu werden in den bekannten Verfahren die primären Bildinformationen mit den zusätzlichen Informationen auf manuelle Anwahl überlagert oder in einem weiteren separaten Anzeigebereich, sozusagen Seite-an-Seite bereitgestellt. In einem Bildausschnitt wird der eigentliche Bildstapel, beispielsweise der CT-Aufnahme dargestellt und daneben kann in einem weiteren Bildausschnitt die Zusatzinformation, beispielsweise die Ventilation, dargestellt werden. Dazu können die Anzeigebereiche hinsichtlich des Bildausschnitts synchronisiert dargestellt werden. Diese Varianten weisen den Nachteil auf, dass jeweils das Bereitstellen der zusätzlichen Informationen manuell an- und abgewählt werden muss und das in der separaten Darstellung der Bezug zu den jeweiligen angezeigten Informationen verloren geht, da der Betrachter zwischen den Bildausschnitten zur Betrachtung hin- und herwechseln muss. Dies erschwert die Korrelation der in den beiden Bildausschnitten wiedergegebenen Informationen, insbesondere zwischen den Anzeigepositionen der Informationen. Beispielsweise kann in der Ventilationskarte ein Defekt ersichtlich sein und diese Position muss in dem anderen Bildausschnitt, der eigentlichen CT-Aufnahme, gefunden werden. Die Alternative zu dieser Betrachtungsweise stellt die Fusion der beiden Bildausschnitte dar, dass letztendlich das Bild der CT-Aufnahme mit den primären Informationen, auf dem die eigentliche Diagnose durchgeführt wird, beispielsweise mit der Ventilationskarte überlagert wird. Allerdings wird durch die Zusatzinformation nicht der eigentliche diagnostische Nutzen dargestellt, wodurch der Betrachter die Überlagerung manuell ein- und ausschalten muss, da die Zusatzinformation nur einen Hinweis an den Betrachter bzw. Befunder gibt, wo befindet sich ein Bereich der genauer betrachtet werden sollte. Somit kann in der überlagerten Darstellung nicht der eigentliche Bildausschnitt, der zur Befundung notwendig ist, betrachtet werden. Diesbezüglich ist ein Wechsel der Darstellung durch ein Ein- und Ausschalten notwendig, der in Komplexität und Aufwand zunimmt, je detaillierter der Bildausschnitt der CT-Aufnahme und der Anzahl der Zusatzinformationen ist.

Es ist daher Aufgabe der vorliegenden Erfindung ein Verfahren und ein System zur Berechnung einer Ausgabe von einem durch einen Tomographen bereitgestellten scrollbaren Bildstapel, der mit zusätzlichen Information versehen ist, bereitzustellen, so dass die Ausgabe eine verbesserte Darstellung mit reduzierter Komplexität beinhaltet. Insbesondere soll hierbei das eigentliche Befunden der Bildausschnitte, beispielsweise das Scrollen der Bildausschnitte durch den Betrachter, für das Berechnen einer Ausgabe berücksichtigt werden.

Diese Aufgabe wird erfindungsgemäß durch ein Verfahren gemäß dem Anspruch 1, eine Recheneinheit gemäß dem Anspruch 12 und ein Computerprogrammprodukt gemäß dem Anspruch 14 gelöst.

Die Erfindung schafft demnach gemäß einem ersten Aspekt ein computerimplementiertes Verfahren zur Berechnung einer Ausgabe von einem durch einen Tomographen bereitgestellten scrollbaren Bildstapel umfassend eine Vielzahl von erzeugten Schnittbildern eines zu untersuchenden Gewebes, mit den Schritten:
Empfangen eines scrollbaren Bildstapels von einem Tomographen;
Berechnen der Ausgabe für eine Anzeigeeinheit, wobei die Ausgabe ein primäres Bild umfasst, das zum Darstellen des empfangenen Bildstapels bestimmt ist und wobei die Ausgabe ein sekundäres Bild mit Zusatzinformationen umfasst, wobei das sekundäre Bild nach Empfang eines Ein- und/oder Ausblendesignals in überlagerter Form über dem primären Bild zur Anzeige gebracht wird, wobei die überlagerte Form ein Überlagern der Grauwerte des primären Bildes mit den Grauwerten des sekundären Bildes durch eine Mischung der Grauwerte umfasst, wobei die Mischung in Abhängigkeit von einer Geschwindigkeit eines Blätterns durch den Bildstapel erfolgt.

Im Sinne der vorliegenden Erfindung ist unter einem Bildstapel ein berechnetes Bild eines Transversalschnittes durch ein Untersuchungsobjekt, welches ein zu untersuchendes Gewebe ist, zu verstehen. Der Transversalschnitt umfasst eine Mehrzahl angrenzender Schnitte, die unterschiedliche Schichten des Untersuchungsobjektes darstellen. Die Schnitte stellen in einer ersten Näherung die in diesem Bereich des Untersuchungsobjektes vorherrschende Dichte dar. Somit stellt der Bildstapel eine in einer Sortierung befindliche Anzahl von Schichten des Untersuchungsobjektes dar.

Zudem ist im Sinne der vorliegenden Erfindung unter einem scrollbaren Bildstapel eine zweidimensionale Darstellung von einer Mehrzahl an axialen Schichten zu verstehen, die durch ein Scrollen durch die Mehrzahl der axialen Schichten betrachtet werden kann, wobei das Scrollen in zwei zueinander gegensätzliche Richtungen erfolgen kann und jeweils eine vorherige oder folgende axiale Schicht, entsprechend der Scrollrichtung, von einer aktuell angezeigten axialen Schicht zur Anzeige bringt.

Der vorliegenden Erfindung liegt die Kenntnis zu Grunde, dass ein Bedarf an einer verbesserten Berechnung einer Ausgabe von einem durch einen Tomographen bereitgestellten scrollbaren Bildstapel besteht, beispielsweise zum Erzielen verbesserter Untersuchungs- und/oder Diagnoseergebnisse mit einer geringeren Fehlerquote. Zudem kann das Durchblättern des Bildstapels effizienter durchgeführt werden ohne, dass ein Informationsverlust (z.B. durch Überdeckung von Bildinformation) in Kauf genommen werden muss.

Die Darstellung in bekannten Stand der Technik Verfahren kann dazu führen, dass das primäre Bild dauerhaft von sekundären Informationen überlagert wird. Wenn notwendig, muss der Betrachter diese Information dann explizit ausblenden und wieder einblenden. Ist die Zusatzinformation insbesondere nützlich, wenn es um die Lokalisierung bestimmter Bildbestandteile während des Durchblätterns des Bildstapels geht, so kann in vorteilhafter Weise durch die vorliegende Erfindung die Zusatzinformation genau dann eingeblendet werden, wenn sie benötigt wird bzw. genau dann ausgeblendet werden, wenn die Anzeige der Zusatzinformation nicht mehr benötigt wird. Ein explizites oder manuelles Ein- und Ausblenden durch den Betrachter entfällt in diesem Fall.

In vorteilhafter Weise können Annotationen, die von einem CAD-Verfahren berechnet wurden und auf bestimmte Anomalien im Bildausschnitt hinweisen sollen, während des Durchblätterns des Bildstapels zur Ausgabe gebracht werden und den Betrachter auf einen entsprechenden Fund hinweisen. In vorteilhafter Weise wird in einem statischen Fall oder entsprechend der Konfiguration, bei einem langsameren Blättern nur das Primärbild dargestellt, um eine entsprechende Befundung durch den Betrachter, beispielsweise einen Radiologen, nicht zu beeinträchtigen. Zudem wird dem Betrachter eine Referenz zwischen der Zusatzinformation und dem Bildstapel bzw. zwischen der Zusatzinformation und einem Schnittbild des Bildstapels bereitgestellt.

Vorteilhafte Ausführungsformen und Weiterbildungen ergeben sich aus den Unteransprüchen, sowie aus der Beschreibung unter Bezugnahme auf die Figuren.

Im Folgenden werden die in dieser Anmeldung verwendeten Begrifflichkeiten näher erläutert.

In einer vorteilhaften Ausführungsform ist der Tomograph ein Magnetresonanztomograph. Ein Magnetresonanztomograph wird zur Durchführung einer Magnetresonanztomographie (MRT) eingesetzt. Die Magnetresonanztomographie ist ein bildgebendes Verfahren, das vor allem in der medizinischen Diagnostik zur Darstellung von Struktur und Funktion der Gewebe und/oder Organe eines menschlichen oder tierischen Körpers eingesetzt wird. Mit dem MRT können Schnittbilder des menschlichen oder tierischen Körpers erzeugt werden, die eine Beurteilung des Gewebes und der Organe bzw. Organgveränderungen aufgrund von Krankheiten erlauben. Das MRT basiert auf von einem Magnetresonanztomographiesysytem erzeugten Magnetfeldern, sowie magnetischen Wechselfeldern im Radiofrequenzbereich, mit denen bestimmte Atomkerne im Körper resonant angeregt werden, wodurch ein elektrisches Signal in Form einer Spannung solange abgegeben wird, bis die Anregung abgeklungen ist. Die Gewebearten unterscheiden sich hinsichtlich ihres molekularen Aufbaus, was zu einem Unterschied in dem elektrischen Signal und einem entsprechenden Bild bzw. Bildstapel führt.

In einer weiteren vorteilhaften Ausführungsform ist der Tomograph ein Computertomograph. Computertomographen basieren auf dem Prinzip der Röntgenstrahlung, bei denen ein Computer eingesetzt wird, um aus den Absorptionswerten von durch den Körper tretenden Röntgensignalen errechnete Schnittbilder zu erzeugen. Durch eine rechnerbasierte Auswertung einer Vielzahl aus verschiedenen Richtungen aufgenommener Röntgenaufnahmen eines Objektes werden die digitale Schnittbilder erzeugt. Die Schnittbilder werden in einem Bildstapel bereitgestellt.

In einer weiteren vorteilhaften Ausführungsform wird das Ein- oder Ausblendsignal während eines Blätterns durch den scrollbaren Bildstapel bereitgestellt. Unter einem Blättern im Sinne der vorliegenden Erfindung ist die Ausgabe eines folgenden bzw. vorherigen Schnittbildes des scrollbaren Bildstapels zu verstehen, so dass jedes separate Schnittbild betrachtet werden kann. Während des Blätterns wird ein entsprechendes Einblendesignal bereitgestellt, womit das sekundäre Bild mit den Zusatzinformationen in überlagerter Form über dem primären Bild zur Anzeige gebracht wird. Insbesondere kann das Ein- und Ausblendesignal für eine vorkonfigurierbare Menge von Schnittbildern oder sogar für jedes einzelne Schnittbild bereitgestellt und zur Berechnung einer veränderten Ausgabe (mit primärem und sekundärem Bild) verarbeitet werden.

In einer weiteren vorteilhaften Ausführungsform wird das Ein- oder Ausblendsignal während eines Vergrößerns oder Verkleinerns des primären Bildes bereitgestellt. In vorteilhafter Weise kann das Ein- oder Ausblendsignal durch ein Vergrößern oder Verkleinern (zooming) eines Bildausschnittes des primären Bildes bereitgestellt werden. Somit kann die Ausgabe des zu untersuchenden Gewebes ohne überlagernde Zusatzinformation beurteilt werden.

In einer weiteren vorteilhaften Ausführungsform wird das Ein- oder Ausblendsignal während eines Verschiebens (panning) des primären Bildes bereitgestellt. In vorteilhafter Weise werden die Zusatzinformationen des sekundären Bildes durch ein Verschieben des primären Bildes in überlagerter Form über dem primären Bild zur Anzeige gebracht. Somit kann die Ausgabe des zu untersuchenden Gewebes ohne überlagernde Zusatzinformation beurteilt werden.

In einer weiteren vorteilhaften Ausführungsform wird das Ein- oder Ausblendsignal in Abhängigkeit von der Geschwindigkeit des Blätterns, Vergrößerns oder Verkleinerns und/oder Verschiebens bereitgestellt. In vorteilhafter Weise kann das Überlagern des primären Bildes an die entsprechenden Ein- und Ausblendsignale angepasst werden und ein feineres Ansprechen der Überlagerung des primären Bildes mit dem sekundären Bild ermöglichen. Beispielsweise erfolgt somit nicht bei jedem durchgeführten Vergrößern oder Verkleinern ein Überlagern des primären Bildes durch das sekundäre Bild, sondern erst, wenn durch einen entsprechenden Geschwindigkeitssensor eine bestimmte und/oder konfigurierte Geschwindigkeit für das Vergrößern oder Verkleinern erfasst worden ist.

In einer weiteren vorteilhaften Ausführungsform wird das Ein- oder Ausblendsignal in einer Multiplane Rekonstruktionsdarstellung (MPR) während einer Änderung der Lage des aktuellen Schnittbildes im Raum durch Rotation oder Krümmungsänderung bereitgestellt. In vorteilhafter Weise kann das sekundäre Bild in überlagerter Form über dem ersten primären Bild zur Anzeige gebracht werden, wenn eine Änderung der Lage des aktuellen Schnittbildes im Raum erfasst worden ist, was davon vermuten lässt, dass weiteren Detailinformationen gewünscht sind und dementsprechend zur Anzeige gebracht werden.

In einer weiteren vorteilhaften Ausführungsform wird ein Ausblendsignal automatisch - und damit vorzugsweise ohne weitere Benutzerinterkation - in einer konfigurierten Zeitdauer nach dem Erzeugen des Einblendsignals (z.B. blättern) bereitgestellt. In vorteilhafter Weise kann das überlagernde sekundäre Bild mittels einer konfigurierbaren Ausblendzeit ausgeblendet werden. Beispielsweise kann die Ausblendzeit so konfiguriert werden, dass ein Ausblenden in dynamischen Schritten (fließend - "fade out"), z.B. Abschwächen der Überlagerung bis zur Transparenz erfolgt und einen verbesserten Vergleich zwischen dem primären Bild und dem sekundären Bild ermöglicht. Die Ausblendzeit kann beispielsweise in einem Bereich von 10ms bis 5s, bevorzugt in einem Bereich 20ms bis 2,5s, besonders bevorzugt in einem Bereich von 20ms bis 1s liegen. Die Ausblendzeit ist durch den Betrachter konfigurierbar. Zu allen anderen Zeitpunkten wird nur die primäre Bildinformation dargestellt.

In einer Ausführungsform wird die Ausblendzeit automatisch auf Basis der Geschwindigkeit des Blätterns, Vergrößerns oder Verkleinerns und/oder Verschiebens konfiguriert.

In einer alternativen Ausführungsform wird das sekundäre Bild so lange in der überlagerten Form über dem primären Bild zur Anzeige gebracht, bis die Ausblendzeit abgelaufen ist. Nach Ablauf der Ausblendzeit wird das sekundäre Bild ausgeblendet.

In einer weiteren vorteilhaften Ausführungsform wird das Primärbild in einer anderen CT-Werte Bereich umfassenden Fensterung eingeblendet. In vorteilhafter Weise wird die Anatomie im Überblick besser erkennbar gemacht.

In einer weiteren vorteilhaften Ausführungsform umfassen die Zusatzinformationen wenigstens Landmarken. Eine Landmarke stellt eine Annotation dar, die den Charakter der Erklärung bzw. Ergänzung hat. Eine Landmarke bezeichnet beispielsweise einen besonderen Punkt in einem Schnittbild, in einer zusammenhängenden Betrachtung von mehreren Schnittbildern oder in einem Bildstapel, dargestellt durch z.B. Kreuze, Punkte, Pfeile, Einkreisung, Markierungen, die manuell oder automatisch schnell wiedergefunden werden können bzw. schnell erkannt werden und eine Abbildung mehrerer Schnittbilder aufeinander gestatten. Zudem stellen Landmarken eine Information über die Position dar und können eine Beschreibung für das Auffinden von bestimmten Bereichen enthalten. Landmarken können punktförmige, lokalisierbare Merkmale im Bild anzeigen, beispielsweise anatomische Strukturen oder Läsionen. Die Landmarken können mit einem Bezeichner zur Beschreibung versehen werden.

In einer weiteren vorteilhaften Ausführungsform umfassen die Zusatzinformationen wenigstens Konturen. Konturen können die Grenzen eines bestimmten Volumens im Bildstapel zu dessen Umgebung markieren bzw. abgrenzen, beispielsweise Organteile, Organe, Organsysteme, Körperteile, Veränderungen (insbesondere Tumore), Implantate. Die Kontur des Volumens kann beispielsweise in einem Therapieplanungssystem verwendet werden, um abschätzen zu können, wieviel Dosis beispielsweise bei einer Strahlungstherapie während der Therapie dieses Organ abbekommt. Alternativ kann mittels einer Kontur ein Tumor und dessen Größe bzw. Veränderung exakt kontrolliert werden, da das Volumen und/oder die Grenze exakt eingezeichnet werden kann.

In einer vorteilhaften Ausführungsform können die Landmarken und Konturen in einer Speichereinheit gespeichert werden und für eine weitere Untersuchungen als Referenz und/oder Vergleich verwendet werden. Die Speichereinheit umfasst beispielsweise eine Festplatte (HDD), einen Direktzugriffsspeicher (RAM), einen Flash-Speicher und/oder eine externe Speichervorrichtung, zum Beschreiben eines tragbaren Datenspeichers.

In einer weiteren vorteilhaften Ausführungsform umfassen die Zusatzinformationen wenigstens Bezeichner/Klassifizierungsinformationen. Die Bezeichner/Klassifizierungsinformationen können verwendet werden, um erkannte Veränderungen, beispielsweise durch einen Tumor als gutartig oder bösartig, z.B. durch Farbmarken oder Textmarken zu kennzeichnen. Die Bezeichner/Klassifizierungen können beispielsweise für das Anlernen neuronaler Netze verwendet werden, da diese eine erkannte Veränderung anzeigen und eine Klassifizierung dieser Veränderung bereitstellen. Durch das Anlernen der Bezeichner/Klassifizierungsinformationen in einem neuronalen Netz kann eine automatische Detektion von Veränderungen erreicht werden. Die Bezeichner/Klassifizierungsinformationen stellen durch das überlagerte Darstellen auf dem primären Bild zudem eine Zusatzinformation dar.

In einer weiteren vorteilhaften Ausführungsform umfassen die Zusatzinformationen wenigstens einen weiteren Bildstapel, der sich aus verschiedenartigen Rekonstruktionen des gleichen Aufnahmedatensatzes und/oder weiterer Aufnahmedatensätze ergibt. Rekonstruktionen können sich dabei insbesondere durch Artefaktkorrekturverfahren (Strahlhärtungskorrektur und/oder Metallartefaktkorrektur) und/oder Rekonstruktionskerne und/oder Regularisierungsparameter unterscheiden. In vorteilhafter Weise kann innerhalb der Rekonstruktion ein Einfluss auf die resultierende Bildschärfe genommen werden. Beispielsweise kann ausgewählt werden, dass ein Bild möglichst sehr scharf rekonstruiert wird, was aber dazu führen kann, dass das Bild gleichzeitig ein hohes Rauschen beinhaltet. Alternativ kann ein Bild mit geringem Rauschen gefordert sein, weil kleine Dichteunterschiede untersucht werden sollen. In vorteilhafter Weise kann im Rahmen der Rekonstruktion darauf Einfluss genommen werden, wie der resultierende Bildeinfluss ist, womit Bilder für den entsprechenden Zweck und Einsatz bereitgestellt werden können. Beispielsweise kann für eine Weichgewebebefundung ein eher unscharfes aber rauschfreies Bild von Vorteil sein, während bei der Beurteilung von feinen Brüchen an Knochen oder Knochenkanten eine scharfe Rekonstruktion vorteilhaft ist.

Mittels der Artefaktkorrekturverfahren können Störungen durch Implantate (Metall) in den einzelnen Schnittbildern des Bildstapel behoben werden. Allerdings haben Artefaktkorrekturverfahren den Nachteil, dass eventuell krankhafte Strukturen durch die Korrektur ebenfalls "weg korrigiert" (und damit entfernt) werden, so dass diese nicht mehr befundet werden können. In vorteilhafter Weise können in einem primären Bild ein Bildstapel ohne Artefaktkorrekturverfahren und in einem sekundären Bild ein Bildstapel mit Artefaktkorrekturverfahren in einer überlagerten Form zu Anzeige gebracht werden, womit eventuell krankhafte Strukturen besser identifiziert werden. Eine Verifikation der dargestellten Informationen wird vereinfacht.

In einer weiteren vorteilhaften Ausführungsform umfassen die Zusatzinformationen wenigstens einen weiteren Bildstapel, der sich aus einer Nachverarbeitung des primären Bildes und/oder anderer Bilder ergibt. In vorteilhafter Weise kann die Zusatzinformation weitere Bildstapel umfassen, die z.B. durch Bildfilter (Rauschunterdrückung, Kantenvorhebung), Punkttransformation (z.B. Histogramm-Equalisierung, Histogramm-Angleich) nachbearbeitet sind. Eine Nachverarbeitung kann in vorteilhafter Weise auch zu Annotationen (Landmarken, Konturen, Textmarken) führen. Diese können in einem Bildstapel überführt werden, der diese Annotationen als Rastergrafik enthält, wobei die Bildbereiche, die kein Annotationsgrafikelement enthalten, vollständig transparent gehalten werden. In einer alternativen Ausführungsform können die Bildwerte eines des ursprünglich der Nachverarbeitung zugeführten Bildstapels oder ein Bildwert, der sich aus einer Kombination der ursprünglichen Bildstapel ergibt, eingetragen werden, so dass die Annotationsgrafikelemente diesen überführten Bildwert überschreiben oder überlagern.

In einer weiteren vorteilhaften Ausführungsform wird für die Zusatzinformationen die kontinuierlich das primäre Bild überlagern, während eines Blätterns durch den Bildstapel ein Ausblendsignal bereitgestellt. Es wird das sekundäre Bild mit den Zusatzinformation dauerhaft in überlagerter Form über dem primären Bild zur Anzeige gebracht und während dem Blättern wird ein Ausblendsignal bereitgestellt, mit dem das sekundäre Bild ausgeblendet wird.

Insbesondere kann durch die Mischung der Grauwerte das sekundäre Bild den primären Bildinhalt (angezeigter Inhalt des primären Bildes) teilweise oder ganz überdecken. In vorteilhafter Weise können das primäre Bild und das sekundäre Bild als ein primäres Grauwertbild und ein sekundäres Grauwertbild dargestellt werden, in dem die Farbwerte ausgeblendet werden. Die Grauwertbilder stellen letztendlich nur eine eindimensionale Form der Bilder dar, da mit einem CT nur der effiziente Schwächungskoeffizient bestimmt werden kann und für eine Farbdarstellung zusätzliche und umfangreichere Berechnungen und somit mehr Rechenleistung erforderlich sind.

In einer Ausführungsform kann für das primäre Grauwertbild ein Bild verwendet werden, auf das keine Artefaktkorrekturen angewendet wurden und für das sekundäres Bild mit Zusatzinformationen kann das Bild, das mit Artefaktkorrekturen rekonstruiert wurde, verwendet werden. Während einem Durchscrollen kann beispielsweise das Bild ohne Artefakte dargestellt werden. Wenn nicht mehr durch das Bild gescrollt wird, wird das Bild mit den Artefakten dargestellt. In vorteilhafter Weise sind somit die Unterschiede zwischen dem ersten und dem zweiten Grauwertbild ersichtlich.

Die Mischung kann somit entsprechend einer Präferenz des Betrachters charakterisiert werden. Bei einem schnelleren Blättern werden die Zusatzinformationen beispielsweise umso stärker zur Anzeige gebracht, bis zu dem Punkt, dass ab einer bestimmten Blättergeschwindigkeit die Zusatzinformation den primären Bildgehalt vollständig überlagert. Die Mischung der Grauwerte kann nach dem Beenden des Blätterns durch den Bildstapel für einen endlichen Zeitraum in dem Zustand beibehalten werden, in dem die Mischung sich während des Blätterns befand.

In einer alternativen Ausführungsform kann ein kontinuierliches Ausblenden der Grauwerte des sekundären Bildes mit den Zusatzinformationen bis zum Ende des Ausblendzeitraumes stattfinden. Die Zeitdauer des Ausblendezeitraumes und das Ausblendverhalten kann dabei durch den Blättervorgang direkt oder indirekt beeinflusst werden.

In einer weiteren vorteilhaften Ausführungsform erfolgt die Mischung der Grauwerte durch eine lineare Kombination umfassend einen ersten Koeffizienten und einen zweiten Koeffizienten, die den Anteil des primären Bildes und des sekundären Bildes bestimmen. In vorteilhafter Weise kann somit berechnet werden, welcher Grauwert des primären Bildes oder des sekundären Bildes dargestellt wird. Der erste Koeffizient beschreibt den Anteil des Primärbildes und der zweite Koeffizient beschreibt den Anteil des sekundären Bildes mit der Zusatzinformation in der Mischung der Grauwerte. Insbesondere bei einer vollständigen Überlagerung durch das sekundäre Bild mit der Zusatzinformation weist der erste Koeffizient einen Betrag gleich Null auf. In den Zeiträumen, in denen nur das primäre Bild angezeigt wird, weist der erste Koeffizient einen Betrag gleich Eins und der zweite Koeffizient einen Betrag gleich Null auf. Insbesondere wird eine gewichtete Summe von Grauwerten gebildet, wobei die Grauwertbilder des primären und sekundären Bildes als Vektoren dargestellt sind.

Das primäre Grauwertbild entspricht dem Vektor 1 und das sekundäre Grauwertbild dem Vektor 2. Die Mischung erfolgt durch eine lineare Kombination der Vektoren. In diesem Fall wird der Vektor 1 mit einem skalaren Faktor multipliziert und der Vektor 2 mit einem weiteren skalaren Faktor multipliziert. Das gemischte Grauwertbild ergibt sich somit aus der Summe der beiden skalierten Vektoren. In vorteilhafter Weise kann somit aus zwei Grauwertbildern ein Bild umfassend Grauwerte dargestellt werden, das eine dynamische Mischung der Grauwerte ermöglicht.

In einer weiteren Ausführungsform kann eine zeitlich unveränderliche Mischung erreicht werden, in dem der erste und der zweite Koeffizient zeitlich unveränderlich im Anzeigeraum während des Blätterns durch den Bildstapel gewählt wird. Die Koeffizienten können beispielsweise durch den Betrachter festgelegt werden.

Die Mischung erfolgt in Abhängigkeit der Scrollgeschwindigkeit.

Diesbezüglich sind die Koeffizienten auch geschwindigkeitsabhängig ausgebildet.

In einer weiteren Ausführungsform kann ein kontinuierliches Ausblenden des sekundären Bildes erfolgen. Diesbezüglich kann ausgehend von den zuletzt - während des Blätterns - gewählten Koeffizienten z.B. ein linearer Verlauf im Ausblendezeitraum angenommen werden, in dem der erste Koeffizient wieder den Wert Eins und der zweite Koeffizient wieder den Wert Null annimmt.

In einer weiteren Ausführungsform kann der Zeitraum des Ausblendens durch eine mathematische Funktion bestimmt werden, z.B. durch eine Potenzfunktion, ein Polynom, eine Exponentialfunktion, eine trigonometrische Funktion, sowie deren Kombination und Umkehrung. Insbesondere soll ein stetiger (womöglich auch stetig differenzierbarer) Übergang zwischen dem zuletzt gewählten Zustand im Zeitraum des Blätterns und dem Ausblendezeitraum sowie zwischen dem Ausblendezeitraum und dem Zeitraum erreicht werden, in welchen anschließend nur das primäre Bild dargestellt wird.

In einer Ausführungsform kann die lineare Kombination durch eine affine Kombination ausgeführt werden, bei der die Summe der Koeffizienten zu jedem Zeitpunkt Eins ergeben muss. Das schließt aber nicht aus, dass ein Koeffizient einen negativen Wert aufweisen kann. In einer weiteren Ausführungsform kann die lineare Kombination durch eine Konvexkombination ausgeführt werden. Die Konvexkombination stellt eine affine Kombination mit der Zusatzbedingung dar, dass alle Koeffizienten größer oder gleich Null sind. Dies hat den Vorteil, dass weitere Grauwertbilder bzw. Varianten von Grauwertbildern in die Mischung einbezogen werden können.

In einer weiteren vorteilhaften Ausführungsform umfasst die überlagerte Form ein Überlagern der Grauwerte mit Werten aus dem Farbraum oder mit einem Wert für die Transparenz. Grauwerte eines Grauwertbildes stellen diesbezüglich eine eindimensionale Untermenge im Bereich der Farbwerte dar und können daher in vorteilhafter Weise mit Farbwertbildern kombiniert werden. Die Transparenzinformation ermöglicht diesbezüglich, dass die Zusatzinformation nicht im ganzen Bildbereich zur Überlagerung kommt. In der Regel wird ein Farbwert, beispielsweise durch einen Vektor mit drei Einzelkomponenten dargestellt. Jede dieser drei Einzelkomponenten ist einer der Grundfarben (rot, grün, blau) zugeordnet und gibt die Helligkeit der Grundfarbe in der Mischung an. Eine Mischung der drei Farben kann somit den gesamten Farbraum abdecken. Ohne eine Transparenz lässt sich die Mischung aus Primärbild und Zusatzbild bzw. sekundärem Bild ebenfalls als lineare Kombination von Bildwertvektoren mittels eines ersten und zweiten Koeffizienten in Analogie entsprechend den Ausführungsformen zur Mischung von Grauwertbildern ausführen. In einer weiteren Ausführungsform kann jedem Bildwert im primären Bild und im sekundären Bild ein zusätzlicher Transparentwert zugeordnet werden. Der Transparenzwert weist beispielsweise einen Wert größer oder gleich Null und kleiner oder gleich Eins auf. In einer Mischung muss dieser Transparenzwert mit dem jeweils ersten oder zweiten Koeffizienten kombiniert werden, beispielsweise durch Multiplikation und anschließender Renormierung, um die Summe der sich ergebenden effektiven Koeffizienten auf Eins zu halten und, um z.B. eine affine Kombination zu erreichen.

In einer weiteren Ausführungsform wird die zu überlagernde Zusatzinformation in Form eines zu überlagernden Bildwertes während des Blättervorgangs aus den Bildwerten des primären Bildes und/oder zu vorherigen Zeitpunkten angezeigten und/oder überlagerten Bildwerten des primären Bildes berechnet. Der zu überlagernde Bildwert kann entsprechend der in den vorherigen Ausführungsformen beschriebenen Varianten mit den Bildwerten des primären Bildes gemischt werden, beispielsweise über eine lineare Kombination oder einer Variante der linearen Kombination. Die Berechnung des überlagernden Bildwertes wird durch die Geschwindigkeit des Blätterns in der Verfahrensweise und Parametrierung variiert.

Der überlagerte Bildwert kann beispielsweise eine lineare Kombination des aktuellen Bildwertes mit einer Anzahl der zuvor berechneten Bildwerte sein. Zudem können die Koeffizienten der linearen Kombination durch die Geschwindigkeit des Blätterns beeinflusst werden.

In einer weiteren Ausführungsform kann das sekundäre Bild mit den Zusatzinformationen, welches dauerhaft in überlagerter Form zur Anzeige gebracht wird, während des Blätterns durch den Bildstapel ausgeblendet werden. Diesbezüglich muss ein Koeffizient, der die Überlagerung dieser auszublendenden Zusatzinformation steuert, während des Blätterns einen Wert gleich Null und im statischen Fall einen Wert ungleich Null aufweisen. In vorteilhafter Weise lässt sich die Ausführungsform mit dem Einblenden während des Blätterns kombinieren.

In einer weiteren Ausführungsform kann die Mischung des primären und sekundären Bildes entsprechend den resultierenden zur Anzeige bestimmten Helligkeits- oder Farbwerten erfolgen. Dies hat den Vorteil, dass das primäre Bild als Zusatzinformation eingeblendet wird, aber eine andere Fensterung aufweist. Eine Fensterung legt fest, welcher Ausschnitt der Skala der gemessenen Dichtewerte im Bild Grauwerten von Schwarz bis Weiß zugewiesen werden. So wird der Bildkontrast der zu befundenden Gewebe optimiert. Eine Fensterung wird auch für die Darstellung von Signalintensitäten angewendet. Dies hat den Hintergrund, dass die in einem Bildstapel abgespeicherten Werte nicht immer direkt einem für die Anzeige bestimmten Bildwert bzw. Helligkeitswert entsprechen. Diesbezüglich muss eine Konvertierung dieser Werte in einen Helligkeitswert erfolgen. Beispielsweise wird die in den Bildstapel enthaltene Schwächungsinformation in Hounsfield Einheiten (HU) abgespeichert. Bei der Fensterung wird ein Intervall der HU-Skala ausgewählt, wobei Werte unterhalb der unteren Grenze oder oberhalb der oberen Grenze der entsprechenden Grenze zugeordnet werden. Das Werteintervall wird dann beispielsweise auf ein linear ansteigendes Helligkeitsintervall bzw. ein Graustufenintervall abgebildet. Alternativ kann dieses Werteintervall auf ein helligkeitsveränderliches Farbintervall oder eine beliebige Farbskala abgebildet werden. Alternativ kann beispielsweise die in einer gleichen Skala abgespeicherten Werte, beispielsweise zwei Bildstapel in der HU-Skala, entsprechend gemischt und folgend auf einer Helligkeitsskala abgebildet werden.

In einer alternativen Ausführungsform wird nur ein primäres Bild zur Anzeige gebracht, während ein Blättern eine andere Parametrierung der Darstellung bereitstellt. Die Parametrierung kann dabei kontinuierlich zu Beginn des Blätterns und nach Beenden des Blätterns erfolgen. Beispielsweise kann es während der statischen Anzeige sinnvoll sein, eine Fensterung zu wählen, die spezifisch an den Bereich des Gewebes und die entsprechende Aufgabe angepasst ist. Diese Fensterung kann dazu insbesondere auf ein vergleichsweise kleines darzustellendes Messwert-Intervall eingeschränkt werden (z.B. bei Röntgen-CT: auf ein kleines HU-Intervall). Die Fensterung ist dann sozusagen die Abbildung des darzustellenden Messwertintervalls auf das Bildwertintervall. In vorteilhafter Weise können somit kleine Kontraste im Weichgewebe dargestellt werden. Ferner wird während des Blätterns ein größeres Messwert-Intervall (z.B. insbesondere HU-Intervall) abgebildet, um die Anatomie im Überblick besser darzustellen zu können. Zudem kann die Breite des Messwert-Intervalls von der Geschwindigkeit des Blätterns abhängig sein. Beispielsweise wird das Intervall breiter, je größer die Blättergeschwindigkeit ist.

Bisher wurde die Erfindung in Bezug auf das beanspruchte Verfahren beschrieben. Merkmale, Vorteile oder alternative Ausführungsformen können den anderen beanspruchten Objekten (z.B. der Recheneinheit, dem System oder einem Computerprogrammprodukt) zugeordnet werden und umgekehrt. Mit anderen Worten, der Gegenstand, der in Bezug auf die Recheneinheit beansprucht oder beschrieben wird, kann durch Merkmale verbessert werden, die im Rahmen des Verfahrens beschrieben oder beansprucht werden und umgekehrt. In diesem Fall werden die Funktionsmerkmale des Verfahrens durch Struktureinheiten der Recheneinheit oder des Systems verkörpert und umgekehrt. Im Allgemeinen sind in der Informatik eine Software-Implementierung und eine entsprechende Hardware-Implementierung gleichwertig. So kann beispielsweise ein Verfahrensschritt zum "Speichern" von Daten mit einer Speichereinheit und entsprechenden Anweisungen zum Schreiben von Daten in den Speicher durchgeführt werden. Um Redundanzen zu vermeiden, kann die Recheneinheit zwar auch in den mit Bezug auf das Verfahren beschriebenen alternativen Ausführungsformen verwendet werden, diese Ausführungsformen werden jedoch für die Recheneinheit nicht noch einmal explizit beschrieben. Die Erfindung schafft gemäß einem weiteren Aspekt eine Recheneinheit gemäß dem Anspruch 12.

Die Recheneinheit zur Ausführung der oben genannten Klassifizierungsaufgabe kann als ein Computer, Personal-Computer oder als eine Workstation in einem Computernetzwerk ausgebildet sein und kann eine Verarbeitungseinheit (Prozessor oder Prozessoren), einen Systemspeicher und einen Systembus beinhalten, der verschiedene Systemkomponenten einschließlich des Systemspeichers mit der Verarbeitungseinheit koppelt. Der Systembus kann eine von mehreren Arten von Busstrukturen sein, einschließlich eines Speicherbusses oder einer Speichersteuerung, eines Peripheriebusses und eines lokalen Busses unter Verwendung einer beliebigen Vielzahl von Busarchitekturen. Der Systemspeicher kann einen Nur-Lese-Speicher (ROM) und/oder einen Direktzugriffsspeicher (RAM) beinhalten. Ein grundlegendes Ein-/Ausgabesystem (BIOS), das grundlegende Routinen enthält, die helfen, Informationen zwischen Elementen innerhalb des PCs zu übertragen, z.B. beim Start, kann im ROM gespeichert werden. Die Recheneinheit kann auch ein Festplattenlaufwerk zum Lesen von und Schreiben auf eine Festplatte, ein Magnetplattenlaufwerk zum Lesen oder Schreiben auf eine (z.B. entfernbare) Magnetplatte und ein optisches Plattenlaufwerk zum Lesen oder Schreiben auf eine entfernbare (magnetische) optische Platte wie eine Kompaktplatte oder andere (magnetische) optische Medien beinhalten. Das Festplattenlaufwerk, das Magnetplattenlaufwerk und das (magnetische) optische Laufwerk können über eine Festplattenschnittstelle, eine Magnetplattenantriebsschnittstelle und eine (magnetische) optische Antriebsschnittstelle mit dem Systembus gekoppelt werden. Die Laufwerke und die zugehörigen Speichermedien bieten eine nichtflüchtige Speicherung von maschinenlesbaren Anweisungen, Datenstrukturen, Programmmodulen und anderen Daten für den Computer. Obwohl die hierin beschriebene exemplarische Umgebung eine Festplatte, eine austauschbare Magnetplatte und eine austauschbare (magnetische) optische Platte verwendet, wird der Fachmann es zu schätzen wissen, dass andere Arten von Speichermedien, wie z.B. Magnetkassetten, Flash-Speicherkarten, digitale Videoplatten, Bernoulli-Kassetten, "Random Access Memory" (RAMs), "Read Only Memory" (ROM) und dergleichen, anstelle oder zusätzlich zu den oben vorgestellten Speichervorrichtungen verwendet werden können. Auf der Festplatte, der Magnetplatte, der (magnetisch-) optischen Platte, dem ROM oder dem RAM können mehrere Programmmodule gespeichert werden, wie beispielsweise ein Betriebssystem, ein oder mehrere Anwendungsprogramme, wie das Verfahren zur Berechnung einer Ausgabe und/oder andere Programmmodule und/oder Programmdaten. Ein Benutzer kann Befehle und Informationen in den Computer über Eingabevorrichtungen, wie beispielsweise eine Tastatur und ein Zeigegerät, eingeben. Andere Eingabevorrichtungen wie Mikrofon, Joystick, Spielepad, Satellitenschüssel, Scanner oder dergleichen können ebenfalls enthalten sein. Diese und andere Eingabegeräte werden oft über eine serielle Schnittstelle, die mit dem Systembus gekoppelt ist, mit der Verarbeitungseinheit verbunden. Eingabegeräte können jedoch auch über andere Schnittstellen verbunden werden, wie z.B. über eine parallele Schnittstelle, eine Spiele-Schnittstelle oder einen universellen seriellen Bus (USB). Ein Monitor (z.B. eine GUI) oder eine andere Art von Anzeigevorrichtung kann auch über eine Schnittstelle, wie z.B. einen Videoadapter, an den Systembus angeschlossen werden. Zusätzlich zum Monitor kann der Computer auch andere periphere Ausgabegeräte wie beispielsweise Lautsprecher und Drucker beinhalten.

Die Recheneinheit kann in einer Netzwerkumgebung betrieben werden, die logische Verbindungen zu einem oder mehreren entfernten Computern definiert. Der entfernte Computer kann ein anderer Personal-Computer, ein Server, ein Router, ein Netzwerk-PC, eine Peer-Vorrichtung oder ein anderer gemeinsamer Netzwerkknoten sein und kann viele oder alle der oben beschriebenen Elemente in Bezug auf den Personal-Computer beinhalten. Zu den logischen Verbindungen gehören ein Local Area Network (LAN) und ein Wide Area Network (WAN), ein Intranet und das Internet.

Ferner kann die Recheneinheit als ein System-on-a-ship Design auf einem Mikrocontroller oder programmierbaren Chip, beispielsweise einem ASSIC oder FPGA implementiert sein.

In einer Ausführungsform wird ein System zur verbesserten Untersuchung von einem bildgebenden Tomographen bereitgestellter dynamischer Bildstapel geschaffen.

Demnach wird ein System zur Berechnung einer Ausgabe von einem durch einen Tomographen bereitgestellten scrollbaren Bildstapel, umfassend eine Vielzahl erzeugter Schnittbilder eines zu untersuchenden Gewebes, geschaffen, welches umfasst:
einen Tomographen, ausgebildet zum Bereitstellen eines scrollbaren Bildstapels;
eine Recheneinheit gemäß der vorliegenden Erfindung;
eine Anzeigeeinheit, ausgebildet zum Darstellen der berechneten Ausgabe.

Die Anzeigeeinheit umfasst einen Monitor, ein Display, ein Touch-Display oder ein Anzeigepanel, das über eine Kommunikationsverbindung mit dem System und/oder den Komponenten des Systems kommuniziert. Die Kommunikationsverbindung kann als eine DVI, HDMI, Display-Port-Anschluss, USB, S-Video/Composite Video und/oder über eine Netzwerkverbindung mittels einem Stream erfolgen.

Die Erfindung schafft ferner ein Computerprogramm mit Programmcode für das Ausführen eines Verfahrens nach einem der vorherigen Verfahrensansprüche, wenn das Computerprogramm auf einem elektronischen Gerät ausgeführt wird. Das Computerprogramm kann als Signal per Download bereitgestellt oder in einer Speichereinheit einer tragbaren Vorrichtung mit darin enthaltenem computerlesbarem Programmcode gespeichert werden, um ein System zur Ausführung von Anweisungen gemäß dem oben genannten Verfahren zu veranlassen. Die Realisierung der Erfindung durch ein Computerprogrammprodukt hat den Vorteil, dass bereits vorhandene elektronische Geräte, beispielsweise Computer, portable Geräte leicht durch Software-Updates verwendet werden können, um wie von der Erfindung vorgeschlagen, eine verbesserte Berechnung einer Ausgabe von einem durch einen Tomographen bereitgestellten scrollbaren Bildstapel zu ermöglichen.

Das Computerprogramm kann in verteilter Weise ausgeführt werden, so dass beispielsweise vereinzelte Verfahrensschritte auf einer ersten Recheneinheit ausgeführt werden und das andere Verfahrensschritte auf einer zweiten Recheneinheit ausgeführt werden.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmalen der Erfindung. Insbesondere wird dabei der Fachmann auch Einzelaspekte als Verbesserung oder Ergänzungen zu der jeweiligen Grundform der vorliegenden Erfindung hinzufügen.

Die vorliegende Erfindung wird nachfolgend anhand der in den schematischen Figuren der Zeichnungen angegebenen Ausführungsbeispiele näher erläutert. Es zeigen dabei:
- Fig. 1: ein Blockdiagramm zur Darstellung einer berechneten Ausgabe von einem durch einen Tomographen bereitgestellten scrollbaren Bildstapel;
- Fig. 2: ein Ablaufdiagramm zur Darstellung eines möglichen Ausführungsbeispiels eines erfindungsgemäßen Verfahrens;
- Fig. 3: ein Blockdiagramm zur Darstellung eines möglichen Ausführungsbeispiels einer erfindungsgemäßen Recheneinheit;
- Fig. 4: ein Blockdiagramm zur Darstellung eines Ausführungsbeispiels eines erfindungsgemäßen Systems.

### Detaillierte Beschreibung der Figuren

Die beiliegenden Zeichnungen sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktionsgleiche, und gleich wirkende Elemente, Merkmale und Komponenten - sofern nichts anderes ausgeführt ist - jeweils mit denselben Bezugszeichen zu versehen.

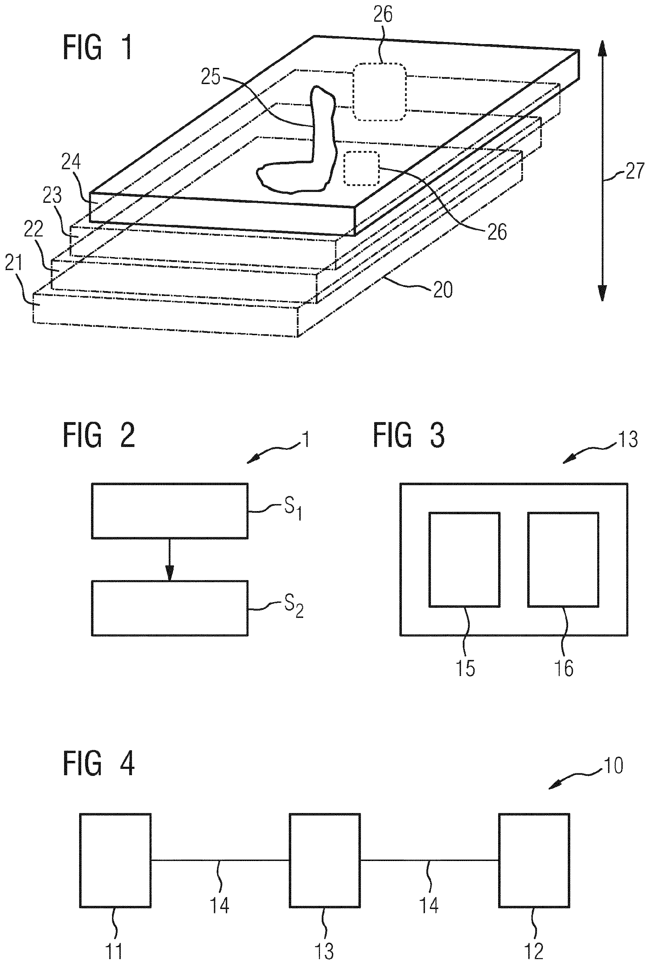

Fig. 1 zeigt ein Blockdiagramm zur Darstellung einer berechneten Ausgabe von einem durch einen Tomographen 11 (siehe Fig. 4) bereitgestellten scrollbaren Bildstapel 20. Der Tomograph 11 kann als ein Magnetresonanztomograph oder als ein Computertomograph ausgebildet sein. Weitere technische Ausführungsformen, Abwandlungen und technische Erneuerungen für Tomographen, die Bilder in einem Bildstapel 20 zur Analyse bereitstellen sind für die vorliegenden Erfindung anwendbar. Ein Bildstapel 20 stellt ein berechnetes Bild eines Transversalschnittes durch ein Untersuchungsobjekt, welches ein zu untersuchendes Gewebe 25 ist, dar. Der Transversalschnitt umfasst eine Mehrzahl an angrenzender Schnitte bzw. Schnittbilder 21, 22, 23, 24, die unterschiedlichen Schichten des Untersuchungsobjektes darstellen. Die Schnittbilder 21, 22, 23, 24 stellen in einer ersten Näherung die in diesem Bereich des Untersuchungsobjektes vorherrschende Dichte dar. Somit stellt der Bildstapel 20 eine in einer Sortierung befindliche Anzahl von Schichten des Untersuchungsobjektes dar. Das Schnittbild 24 stellt beispielsweise das aktive bzw. aktuell dargestellt Schnittbild des Bildstapels 20 dar, welches durch einen Betrachter, beispielsweise durch einen Radiologen zur Befundung betrachtet wird. In dem Schnittbild 24 ist eine Schicht des zu untersuchenden Gewebes 25 dargestellt. Unter einem zu untersuchenden Gewebe 25 ist das Gewebe eines menschlichen oder tierischen Körpers oder Organe eines menschlichen oder tierischen Körpers zu verstehen. Zudem sind in dem Schnittbild 24 in einem sekundären Bild Zusatzinformationen 26 dargestellt. Das sekundäre Bild mit den Zusatzinformationen 26 wird nach einem Empfang eines Ein- oder Ausblendesignals 27 in überlagerter Form über dem primären Bild mit dem Bildstapel 20 umfassend die Schnittbilder 21, 22, 23, 24 zur Anzeige gebracht.

Die Zusatzinformationen 26 umfassen in einem Ausführungsform Annotationen. Die Annotationen umfassen beispielsweise Landmarken, Konturen und/oder Bezeichner/Klassifizierungsinformationen. In einer weiteren Ausführungsform umfassen die Zusatzinformationen 26 einen weiteren Bildstapel 20, der sich aus verschiedenartigen Rekonstruktionen des gleichen Aufnahmedatensatzes und/oder weiter Aufnahmedatensätze ergibt. In einer weiteren Ausführungsform umfassen die Zusatzinformationen 20 Bildstapel, die sich aus einer Nachverarbeitung des primären Bildes und/oder anderer Bilder ergeben.

Gemäß der vorliegenden Erfindung wird eine Ausgabe für eine Anzeigeeinheit 12 (siehe Fig. 4) berechnet. Die Ausgabe umfasst ein primäres Bild, dass zum Darstellen des empfangenen Bildstapels 20 bestimmt ist. Weiterhin umfasst die Ausgabe ein sekundäres Bild mit Zusatzinformationen 26, wobei das sekundäre Bild nach Empfang eines Ein- oder Ausblendesignals 27 in überlagerter Form über dem primären Bild zur Anzeige gebracht wird. Das Ein- oder Ausblendsignal 27 wird durch die Recheneinheit 13 auf Basis eines Eingabesignals des Betrachters bereitgestellt. Das Eingabesignal kann ein Scrollen, Blättern, Verkleinern, Vergrößern, Verschieben, Rotieren und/oder Krümmen umfassen. In einer Ausführungsform kann das Ein- oder Ausblendsignal 27 durch ein Scrollen bzw. Blättern in dem Bildstapel 20 durch die einzelnen Schnittbilder 21, 22, 23, 24 erfolgen. Durch das Scrollen bzw. Blättern wird entsprechend der Scroll- bzw. Blätterrichtung das vorherige Schnittbild oder das nachfolgende Schnittbild aktive dargestellt. Während des Scrollens bzw. Blätterns werden die Zusatzinformationen 26 in überlagerter Form über dem primären Bild zur Anzeige gebracht. In einer Ausführungsform kann das Ein- oder Ausblendsignal 27 durch ein Vergrößern oder Verkleinern (Zooming) oder durch ein Verschieben (Panning) des Bildausschnittes oder durch eine Rotation und/oder Krümmungsänderung des Schnittbildes 21, 22, 23, 24 in einer MPR-Darstellung erzeugt werden. In einer Ausführungsform werden die Zusatzinformationen 26 solange in überlagerter Form über dem primären Bild zur Anzeige gebracht, wie das Ein- oder Ausblendsignal 27 bereitgestellt wird. Wenn das Ein- oder Ausblendsignal nicht mehr verfügbar ist, werden die Zusatzinformationen ausgeblendet. In einer Ausführungsform können die Zusatzinformationen dynamisch innerhalb eines konfigurierbaren Ausblendezeitraums ausgeblendet werden. In einer weiteren Ausführungsform wird die Zeitdauer des Ausblendens durch die Geschwindigkeit der Scroll- bzw. Blätterrichtung bzw. durch die weiteren Eingabesignale bestimmt.

In einer weiteren Ausführungsform umfasst die überlagerte Form ein Überlagern der Grauwerte des primären Bildes umfassend den Bildstapel 20 mit den Grauwerten eines sekundären Bildes umfassend die Zusatzinformationen 26. In der überlagerten Form können die Grauwerte des primären Bildes wenigstens teilweise oder vollständig durch die Grauwerte des sekundären Bildes überdeckt werden. Die Überlagerung erfolgt durch eine Mischung der Grauwerte. In einem Beispiel, das nicht Teil der vorliegenden Erfindung ist, sondern lediglich einem besseren Verständnis dient, ist die Mischung unveränderlich und entsprechend einer Präferenz des Betrachters charakterisiert. Gemäß der vorliegenden Erfindung ist die Mischung konfigurierbar. Die Mischung wird durch das Scrollen bzw. Blättern durch den Bildstapel 20 konfiguriert. In einer Ausführungsform kann die Mischung durch Vergrößern oder Verkleinern des Bildausschnitts oder durch das Verschieben eines Bildausschnittes konfiguriert werden. Gemäß der vorliegenden Erfindung wird die Mischung durch die Geschwindigkeit des Scrollens bzw. Blätterns durch den Bildstapel 20 konfiguriert. In einer Ausführungsform kann die Mischung durch die Geschwindigkeit des Vergrößerns oder Verkleinerns des Bildausschnitts oder durch die Geschwindigkeit des Verschiebens eines Bildausschnittes konfiguriert werden. Die Mischung der Grauwerte kann nach Beenden des Scrollens bzw. Blätterns durch den Bildstapel 20 bzw. durch die weiteren Eingabesignale für einen konfigurierbaren Zeitraum beibehalten werden und anschließend ausgeblendet werden. In einer alternativen Ausführungsform kann die Mischung der Grauwerte über einen konfigurierbaren Zeitraum dynamisch ausgeblendet werden. Der Zeitraum kann über die Geschwindigkeit der Eingabesignale konfigurierbar sein.

In einer Ausführungsform kann die Grauwertmischung durch eine lineare Kombination, in der ein erster Koeffizient den Anteil des primären Bildes und ein zweiter Koeffizient den Anteil des sekundären Bildes bestimmt, stattfinden. Die lineare Kombination kann als eine affine Kombination oder durch eine Konvexkombination ausgeführt werden.

In einer weiteren Ausführungsform können die Bildwerte auch Werte aus dem Farbraum, einschließlich der Transparenz annehmen. Dies hat den Vorteil, dass beispielsweise für das Anzeigen einer Kontur einer Lunge die entsprechenden Werte des primären Bildes überlagert werden können und somit Erkenntnisse über die Belüftung an den entsprechenden Stellen der Lunge sichtbar werden und z.B. das Volumen bestimmter Bereiche der Lunge während des Ein- und Ausatmens bestimmt werden kann.

In einer alternativen Ausführungsform wird das sekundäre Bild mit den Zusatzinformationen 26 dauerhaft zur Anzeige gebracht und kann durch das Ein- und Ausblendsignal 27, insbesondere durch ein Ausblendsignal ausgeblendet werden. Das Ausblendsignal wird auf Basis des Eingabesignals bereitgestellt. Fig. 2 zeigt ein Ablaufdiagramm zur Darstellung eines möglichen Ausführungsbeispiels eines erfindungsgemäßen Verfahrens. Das Verfahren 1 umfasst bei dem dargestellten Ausführungsbeispiel mehrere Schritte. In einem ersten Schritt S1 wird von einem Tomographen 11 (siehe Fig. 4) ein scrollbarer Bildstapel 20 (siehe Fig. 1) empfangen.

In einem zweiten Schritt S2 erfolgt die Berechnung der Ausgabe für eine Anzeigeeinheit 12 (siehe Fig. 4), wobei die Ausgabe ein primäres Bild umfasst, dass zum Darstellen des empfangenen Bildstapels 20 bestimmt ist und die Ausgabe ein sekundäres Bild mit Zusatzinformationen 26 umfasst, wobei das sekundäre Bild nach Empfang eines Ein- oder Ausblendesignals 27 (siehe Fig. 1) in überlagerter Form über dem primären Bild zur Anzeige gebracht wird. Das sekundäre Bild wird in überlagerter Form über dem primären Bild auf einer Anzeigeeinheit 12 zur Anzeige gebracht.

Fig. 3 zeigt ein Blockdiagramm zur Darstellung eines möglichen Ausführungsbeispiels einer erfindungsgemäßen Recheneinheit.

In Fig. 3 bezeichnet Bezugszeichen 13 eine Recheneinheit. Die Recheneinheit 13 umfasst eine Kommunikationsschnittstelle 15 und eine Verarbeitungseinheit 16. Die Recheneinheit 13 kann, wie bereits ausgeführt, als ein Computer, Personal-Computer, Workstation, als ein Server, oder als ein Computer in einem Serververbund (Cloud) die über ein Kommunikationsnetzwerk, beispielsweise ein lokales Netzwerk oder das Internet kommunizieren, ausgebildet sein.

Die Kommunikationsschnittstelle 14 ist ausgebildet einen scrollbaren Bildstapels 20 von einem Tomographen zu empfangen und die durch die Verarbeitungseinheit 15 berechnete Ausgabe an eine Anzeigeeinheit 12 (siehe Fig. 4) bereitzustellen.

Die Verarbeitungseinheit 16 ist ausgebildet eine Ausgabe für eine Anzeigeeinheit 12 zu berechnen, wobei die Ausgabe ein primäres Bild umfasst, dass zum Darstellen des empfangenen Bildstapels 20 (siehe Fig. 1) bestimmt ist und die Ausgabe ein sekundäres Bild mit Zusatzinformationen 26 (siehe Fig. 1) umfasst, wobei das sekundäre Bild nach Empfang eines Ein- oder Ausblendesignals 27 in überlagerter Form über dem primären Bild zur Anzeige gebracht wird.

Fig. 4 zeigt ein Blockdiagramm zur Darstellung eines Ausführungsbeispiels eines erfindungsgemäßen Systems.

In Fig. 4 bezeichnet Bezugszeichen 10 das erfindungsgemäße System zur Berechnung einer Ausgabe von einem durch einen Tomographen bereitgestellten scrollbaren Bildstapel. Das System 10 umfasst einen Tomographen 11, beispielsweise einen Magnetresonanztomograph oder ein Computertomograph, eine Recheneinheit 13 zum Berechnen einer Ausgabe eines primären Bildes zum Darstellen des empfangenen Bildstapels 20, die durch ein sekundäres Bild mit Zusatzinformationen 26 in überlagerter Form auf einer Anzeigeeinheit 12 zur Anzeige gebracht wird. Die Komponenten (Tomograph 11, Recheneinheit 13, Anzeigeeinheit 12) des Systems 10 kommunizieren über eine Kommunikationsverbindung 14 miteinander. Das System 10 kann als ein verteiltes System 10 ausgebildet sein, bei dem die Komponenten an verteilten Orten positioniert sind und über die Kommunikationsverbindung 14, beispielsweise über ein lokales Netzwerk (LAN) oder über ein globales Netzwerk (MAN, WAN) mit einander verbunden sind und kommunizieren. In einer alternativen Ausführungsform können die Komponenten sehr zentral zu einander positioniert sein und über serielle oder parallele Kommunikationsverbindungen bzw. Kommunikationsschnittstellen (Ethernet, USB, FireWire, CAN-BUS, RS-485) kommunizieren.

## Patentansprüche

1. Computer-implementiertes Verfahren (1) zur Berechnung einer Ausgabe von einem durch einen Tomographen bereitgestellten scrollbaren Bildstapel (20), umfassend eine Vielzahl von erzeugten Schnittbildern (21, 22, 23, 24) eines zu untersuchenden Gewebes (25), mit den Schritten:
- Empfangen (S1) des scrollbaren Bildstapels (20) von einem Tomographen (11);
- Berechnen (S2) der Ausgabe für eine Anzeigeeinheit (12), wobei die Ausgabe ein primäres Bild umfasst, das zum Darstellen des empfangenen Bildstapels (20) bestimmt ist und wobei die Ausgabe ein sekundäres Bild mit Zusatzinformationen (26) umfasst, wobei das sekundäre Bild nach Empfang eines Ein- oder Ausblendesignals (27) in überlagerter Form über dem primären Bild zur Anzeige gebracht wird, wobei die überlagerte Form ein Überlagern der Grauwerte des primären Bildes mit den Grauwerten des sekundären Bildes durch eine Mischung der Grauwerte umfasst, wobei die Mischung in Abhängigkeit von einer Geschwindigkeit eines Blätterns durch den Bildstapel erfolgt.

2. Verfahren (1) nach Anspruch 1, wobei der Tomograph ein Magnetresonanztomograph oder ein Computertomograph ist.

3. Verfahren (1) nach einem der vorherigen Ansprüche, wobei das Ein- oder Ausblendsignal (27) während des Blätterns durch den scrollbaren Bildstapel (20) bereitgestellt wird.

4. Verfahren (1) nach einem der vorherigen Ansprüche, wobei das Ein- oder Ausblendsignal (27) während eines Vergrößerns oder Verkleinerns des primären Bildes bereitgestellt wird.

5. Verfahren (1) nach einem der vorhergehenden Ansprüche, wobei das Ein- oder Ausblendsignal (27) während eines Verschiebens des primären Bildes bereitgestellt wird.

6. Verfahren (1) nach einem der vorhergehenden Ansprüche, wobei das Ein- oder Ausblendsignal (27) in einer Multiplane Rekonstruktionsdarstellung während einer Änderung der Lage des aktuellen Schnittbildes (24) im Raum durch Rotation oder Krümmungsänderung bereitgestellt wird.

7. Verfahren (1) nach einem der vorherigen Ansprüche, wobei ein Ausblendsignal automatisch in einer konfigurierten Zeitdauer nach dem Erzeugen des Einblendsignals bereitgestellt wird.

8. Verfahren (1) nach einem der vorherigen Ansprüche, wobei die Zusatzinformationen wenigstens umfassen:
- Landmarken,
- Konturen,
- Bezeichner,
- Klassifizierungsinformationen,
- einen Bildstapel, der sich aus verschiedenartigen Rekonstruktionen des gleichen Aufnahmedatensatzes und/oder weiterer Aufnahmedatensätze ergibt, und/oder
- einen Bildstapel, der sich aus einer Nachverarbeitung des primären Bildes und/oder anderer Bilder ergibt.

9. Verfahren (1) nach einem der vorherigen Ansprüche, wobei für diejenigen Zusatzinformationen, die kontinuierlich das primäre Bild überlagern, während des Blätterns durch den Bildstapel (20) ein Ausblendsignal bereitgestellt wird.

10. Verfahren (1) nach einem der vorherigen Ansprüche, wobei die Mischung der Grauwerte durch eine lineare Kombination umfassend einen ersten Koeffizienten und einen zweiten Koeffizienten, die den Anteil des primären Bildes und des sekundären Bildes bestimmen, erfolgt.

11. Verfahren (1) nach einem der vorhergehenden Ansprüche, wobei die überlagerte Form ein Überlagern der Grauwerte mit Werten aus dem Farbraum oder mit einem Wert für die Transparenz umfasst.

12. Recheneinheit (13) mit,
- einer Kommunikationsschnittstelle (14), ausgebildet zum Empfangen eines scrollbaren Bildstapels (20), insbesondere von einem Tomographen; und
- einer Verarbeitungseinheit (15), ausgebildet eine Ausgabe für eine Anzeigeeinheit (12) zu berechnen, wobei die Ausgabe ein primäres Bild umfasst, das zum Darstellen des empfangenen Bildstapels (20) bestimmt ist und wobei die Ausgabe ein sekundäres Bild mit Zusatzinformationen (26) umfasst, wobei das sekundäre Bild nach Empfang eines Ein- oder Ausblendesignals (27) in überlagerter Form über dem primären Bild zur Anzeige gebracht wird, wobei die überlagerte Form ein Überlagern der Grauwerte des primären Bildes mit den Grauwerten des sekundären Bildes durch eine Mischung der Grauwerte umfasst, wobei die Mischung in Abhängigkeit von einer Geschwindigkeit eines Blätterns durch den Bildstapel erfolgt, und wobei
die Kommunikationsschnittstelle (14) ferner ausgebildet ist, die berechnete Ausgabe bereitzustellen.

13. System (10) zur Berechnung einer Ausgabe von einem durch einen Tomographen bereitgestellten scrollbaren Bildstapel 20), umfassend eine Vielzahl erzeugter Schnittbilder (21, 22, 23, 24) eines zu untersuchenden Gewebes (25), umfassend:
- einen Tomographen (11), ausgebildet zum Bereitstellen eines scrollbaren Bildstapels (20);
- eine Recheneinheit nach Anspruch 12;
- eine Anzeigeeinheit (12), ausgebildet zum Darstellen der berechneten Ausgabe.

14. Computerprogrammprodukt mit Programmcode für das Ausführen eines Verfahrens (1) nach einem der vorherigen Verfahrensansprüche, wenn das Computerprogramm auf einem elektronischen Gerät ausgeführt wird.

## Claims

1. Computer-implemented method (1) for calculating an output from a tomographic scrollable image stack (20), including a large number of generated sectional images (21, 22, 23, 24) of a tissue (25) to be examined, having the following steps:
- receiving (S1) the scrollable image stack (20) from a tomography unit (11);
- calculating (S2) the output for a display unit (12), wherein the output includes a primary image that is intended for representing the received image stack (20), and wherein the output includes a secondary image with additional information (26), wherein the secondary image is displayed overlaid on the primary image once an unhide or hide signal (27) is received, wherein the overlaid form includes overlaying on the grayscale values of the primary image the grayscale values of the secondary image, by blending the grayscale values, wherein the blending takes place in dependence on a speed of a leafing through the image stack.

2. Method (1) according to claim 1, wherein the tomography unit is a magnetic resonance imaging tomography unit or a computed tomography unit.

3. Method (1) according to one of the preceding claims, wherein the unhide or hide signal (27) is provided during leafing through the scrollable image stack (20).

4. Method (1) according to one of the preceding claims, wherein the unhide or hide signal (27) is provided during zooming in or out of the primary image.

5. Method (1) according to one of the preceding claims, wherein the unhide or hide signal (27) is provided during panning of the primary image.

6. Method (1) according to one of the preceding claims, wherein the unhide or hide signal (27) is provided in a multiplane reconstruction during a change in the position of the current sectional image (24) in space as a result of rotation or a change in curvature.

7. Method (1) according to one of the preceding claims, wherein a hide signal is provided automatically for a configured duration after generation of the unhide signal.

8. Method (1) according to one of the preceding claims, wherein the additional information includes at least:
- landmarks,
- contours,
- identifiers,
- classification information,
- an image stack that is produced from different types of reconstruction of the same captured data set and/or further captured data sets, and/or
- an image stack that results from post-processing the primary image and/or other images.

9. Method (1) according to one of the preceding claims, wherein a hide signal is provided for the additional information that is continuously overlaid on the primary image during leafing through the image stack (20).

10. Method (1) according to one of preceding claims, wherein the grayscale values are blended by a linear combination including a first coefficient and a second coefficient that determine the proportion of the primary image and the secondary image.

11. Method (1) according to one of the preceding claims, wherein the overlaid form includes overlaying values from the colour spectrum or a value for transparency on the grayscale values.

12. A processor unit (13), having
- a communication interface (14) that is constructed to receive a scrollable image stack (20), in particular from a tomography unit; and
- a processing device (15) that is constructed to calculate an output for a display unit (12), wherein the output includes a primary image intended for representing the image stack (20) that is received, and wherein the output includes a secondary image with additional information (26), wherein the secondary image is displayed overlaid on the primary image once an unhide or hide signal (27) is received, wherein the overlaid form includes overlaying on the grayscale values of the primary image the grayscale values of the secondary image, by blending the grayscale values, wherein the blending takes place in dependence on a speed of a leafing through the image stack, and wherein
the communication interface (14) is further constructed to provide the calculated output.

13. System (10) for calculating an output from a tomographic scrollable image stack (20), including a large number of generated sectional images (21, 22, 23, 24) of a tissue (25) to be examined, including:
- a tomography unit (11) constructed to provide a scrollable image stack (20);
- a processor unit according to claim 12;
- a display unit (12) constructed to show the calculated output.

14. Computer program product with program code for performing a method (1) according to one of the preceding method claims when the computer program is executed on an electronic device.

## Revendications

1. Procédé (1) assisté par ordinateur de calcul d'une sortie d'un empilement (20) d'images pouvant défiler et fourni par un tomodensitomètre, comprenant une pluralité d'images (21, 22, 23, 24) de coupe produites d'un tissu (25) à examiner, comprenant les stades :
- réception (S1) de l'empilement (20) d'images pouvant défiler par un tomodensitomètre (11) ;
- calcul (S2) de la sortie pour une unité (12) d'affichage, la sortie comprenant une image primaire, qui est destinée à représenter l'empilement (20) d'images reçu et la sortie comprenant une image secondaire ayant des informations (26) supplémentaires, dans lequel on met, pour l'affichage, l'image secondaire, après réception d'un signal (27) de diaphragme d'entrée ou de sortie, en une forme superposée sur l'image primaire, la forme superposée comprenant une superposition des valeurs de gris de l'image primaire aux valeurs de gris de l'image secondaire, par un mélange des valeurs de gris, le mélange s'effectuant en fonction d'une vitesse d'un feuilletage dans l'empilement d'images.

2. Procédé (1) suivant la revendication 1, dans lequel le tomodensitomètre est un tomodensitomètre à résonnance magnétique ou un tomodensitomètre assisté par ordinateur.

3. Procédé (1) suivant l'une des revendications précédentes, dans lequel on fournit le signal (27) de diaphragme d'entrée ou de sortie pendant le feuilletage dans l'empilement (20) d'images pouvant défiler.

4. Procédé (1) suivant l'une des revendications précédentes, dans lequel on fournit le signal (27) de diaphragme d'entrée ou de sortie pendant un agrandissement ou un rapetissement de l'image primaire.

5. Procédé (1) suivant l'une des revendications précédentes, dans lequel on fournit le signal (27) de diaphragme d'entrée ou de sortie pendant un décalage de l'image primaire.

6. Procédé (1) suivant l'une des revendications précédentes, dans lequel on fournit le signal (27) de diaphragme d'entrée ou de sortie dans une représentation de reconstruction suivant plusieurs plans, pendant une modification de la position de l'image (24) de coupe en cours dans l'espace, par rotation ou modification de courbure.

7. Procédé (1) suivant l'une des revendications précédentes, dans lequel on fournit un signal de diaphragme de sortie automatiquement dans une durée configurée, après la production du signal de diaphragme d'entrée.

8. Procédé (1) suivant l'une des revendications précédentes, dans lequel les informations supplémentaires comprennent au moins :
- des repères,
- des contours,
- des indicateurs,
- des informations de classement,
- un empilement d'images, qui provient de reconstructions de types différents du même ensemble de données d'enregistrement et/ou d'autres ensembles de données d'enregistrement, et/ou
- un empilement d'images, qui provient d'un retraitement de l'image primaire et/ou d'autres images.

9. Procédé (1) suivant l'une des revendications précédentes, dans lequel, pour les informations supplémentaires, qui se superposent continuellement à l'image primaire, on fournit un signal de diaphragme de sortie pendant le feuilletage de l'empilement (20) d'images.

10. Procédé (1) suivant l'une des revendications précédentes, dans lequel le mélange des valeurs de gris s'effectue par une combinaison linéaire comprenant un premier coefficient et un deuxième coefficient, qui déterminent la proportion de l'image primaire et de l'image secondaire.

11. Procédé (1) suivant l'une des revendications précédentes, dans lequel la forme superposée comprend une superposition des valeurs de gris à des valeurs de l'espace d'image ou à une valeur de la transparence.

12. Unité (13) informatique comprenant,
- une interface (14) de communication constituée pour recevoir un empilement (20) d'images pouvant défiler, notamment d'un tomodensitomètre ; et
- une unité (15) de traitement constituée pour calculer une sortie pour une unité (12) d'affichage, la sortie comprenant une image primaire , qui est destinée à représenter l'empilement (20) d'images reçu et la sortie comprenant une image secondaire ayant des informations (26) supplémentaires, dans laquelle on met, pour l'affichage, l'image secondaire, après réception d'un signal (27) de diaphragme d'entrée ou de sortie, en une forme superposée sur l'image primaire, la forme superposée comprenant une superposition des valeurs de gris de l'image primaire aux valeurs de gris de l'image secondaire, par un mélange des valeurs de gris, le mélange s'effectuant en fonction d'une vitesse d'un feuilletage dans l'empilement d'images, et dans laquelle
l'interface (14) de communication est constituée en outre pour fournir la sortie calculée.

13. Système (10) de calcul d'une sortie d'un empilement (20) d'images pouvant défiler et fourni par un tomodensitomètre, comprenant une pluralité d'images (21, 22, 23, 24) de coupe produites d'un tissu (25) à examiner, comprenant
- un tomodensitomètre (11) constitué pour fournir un empilement (20) d'images pouvant défiler ;
- une unité informatique suivant la revendication 12 ;
- une unité (12) d'affichage constituée pour représenter la sortie calculée.

14. Produit de programme d'ordinateur comprenant un code de programme pour la réalisation d'un procédé (1) suivant l'une des revendications de procédé précédentes, lorsque le programme d'ordinateur est réalisé sur un appareil électronique.
